Europäisches Patentamt

European Patent Office

Office européen des brevets

(19)

(11) Publication number: **0 365 209**
**A2**

## EUROPEAN PATENT APPLICATION

(21) Application number: **89310415.8**

(22) Date of filing: **11.10.89**

(51) Int. Cl.5: **C12N 15/13 , C12P 21/08**

(30) Priority: **17.10.88 US 260558**

(43) Date of publication of application:
**25.04.90 Bulletin 90/17**

(84) Designated Contracting States:
**AT BE CH DE ES FR GB GR IT LI LU NL SE**

(71) Applicant: **Becton Dickinson and Company**
**One Becton Drive**
**Franklin Lakes New Jersey 07417-1880(US)**

(72) Inventor: **Hinton, Robert**
**151 Calderon No. 177**
**Mountain View California(US)**
Inventor: **Oi, Vernon T.**
**1259 Marilyn Court**
**Mountain View California(US)**

(74) Representative: **Ruffles, Graham Keith et al**
**MARKS & CLERK 57-60 Lincoln's Inn Fields**
**London WC2A 3LS(GB)**

(54) Anti-leu 3A amino acid sequence.

(57) The nucleotide and amino acid sequence of Anti-Leu 3a and chimeric and mosaic variants thereof is disclosed.

EP 0 365 209 A2

## ANTI-LEU 3a AMINO ACID SEQUENCE

Field of the Invention

This invention relates to an amino acid sequence for an anti-CD4 antibody and more particularly relates to an amino acid sequence for the anti-CD4 antibody, Anti-Leu 3a, and a chimeric variant thereof.

Background of the Invention

CD4 is an antigen on certain T lymphocytes (i.e., the helper subset) that has a molecular weight of approximately 55 Kd. It is thought to consist of four domains ($V_1$ - $V_4$) that extend from the cell membrane outward in serial fashion.

Recently, the CD4 antigen or CD4$^+$ cells have been implicated in certain immune system diseases ranging from autoimmune diseases, such as rheumatoid arthritis and multiple sclerosis, to AIDS. Treatment of these diseases with anti-CD4 agents (i.e., chemical or biological materials that bind to or block the function of the CD4 antigen) has been suggested. See, e.g., U.S. Pat. No. 4,695,459 and Weber et al., Sci. Amer., 259:101 (1988).

Not all anti-CD4 agents, and in particular not all anti-CD4 monoclonal antibodies, however, have the same effect on the CD4 antigen or CD4$^+$ cells. That is, not all anti-CD4 monoclonal antibodies bind to the same region or epitope on CD4. Where a particular monoclonal antibody binds to CD4 is important in a disease such as AIDS, for example. Sattentau et al., Science, 234:1120 (1986), showed that not all anti-CD4 antibodies would cross-block each other in competitive binding studies with CD4$^+$ cells or would block the binding of HIV to CD4$^+$ cells. Recent work, summarized by Weber et al., supra, suggests that a specific amino acid sequence in the outermost or $V_1$ domain of the CD4 molecule is site where HIV binds to CD4, and suggests that this is the site (or very near to it) where the monoclonal antibody, anti-Leu 3a (Becton Dickinson Immunocytometry Systems, BDIS), binds. Also near this site, but different from the site to which Anti-Leu 3a binds, is the site at which another monoclonal antibody, OKT4a (Ortho Diagnostics), binds.

The ability of Anti-Leu 3a to block HIV binding has been attributed to its structure being the same or nearly the same as a portion of the gp120 region of the HIV virus. The gp120 region of the virus has been shown to bind to CD4. Thus, Anti-Leu 3a has a structure that looks like the HIV binding region on gp120 but lacks the disease carrying properties of the virus itself. As result, the use of Anti-Leu 3a as a vaccine is being attempted. See Matthews et al., Sci. Amer., 259:120 (1988).

Anti-Leu 3a is a mouse monoclonal antibody derived from clone SK3 which was originally described by Evans et al., PNAS, 78:544 (1981). SK3 was derived from hybridization of mouse NS-1 myeloma cells with spleens from BALB/c mice immunized with sheep red blood cell rosettes of human peripheral blood. It is a IgG$_1$ type antibody having a kappa light chain. Because Anti-Leu 3a is a mouse monoclonal antibody, however, its use a therapeutic agent, such as an AIDS vaccine, raises certain problems.

Mouse monoclonal antibodies or in fact any other non-human antibodies will be immunogenic when used as a human therapeutic agent. The mouse monoclonal antibody will be recognized as being "non-self" by the recipient's immune system. Thus, once a mouse monoclonal antibody has been used, anti-mouse antibodies will be formed in the host which then will limit the subsequent effectiveness of the agent in the course of further or subsequent treatment. Accordingly, it would be preferable to use human monoclonal antibody. The preparation of a human antibody, however, raises a number of serious practical and ethical questions.

One alternative method to make the mouse monoclonal antibody less immunogenic while retaining the binding specificity of antibody is to make the antibody "chimeric." In one embodiment of this format, the mouse variable region of the antibody is coupled to the constant region of the same type antibody but from another species or another strain of the same species. In the preferred human therapeutic embodiment, the other species is human. For veterinary purposes, the species to be treated will comprise the other species.

Simply stated, the gene for the mouse variable region is isolated and spliced by appropriate genetic engineering techniques, such as those described in chapters 5 and 6 of Recombinant DNA: A Short Course (Watson et al., eds., 1983), to the gene for the human constant region. The resulting gene then will code for an immunoglobulin having the mouse variable region and human constant region. A vector carrying this new gene then may be placed in a prokaryotic or eukaryotic organism for expression of the immunoglobulin. A specific embodiment for this type of chimeric antibody for expression in eukaryotic organisms is set forth in USSN 644,473 filed August 27, 1984, and for expression in prokaryotic organisms is set forth in USSN 483,457 filed April 8, 1983.

The application USSN 644,473 corresponds with published European Patent Specification 173494. The application USSN 483,457 corresponds with published US Patent 4816567.

Another embodiment of a chimeric format is set forth in UK Pat. Appl. GB 2 188 638 filed March 27, 1986, and also is described in Jones et al., Nature, 321:522 (1986). In this approach, the chimeric antibody is "humanized" by introducing more human sequences into the variable gene region while retaining the specific mouse binding regions.

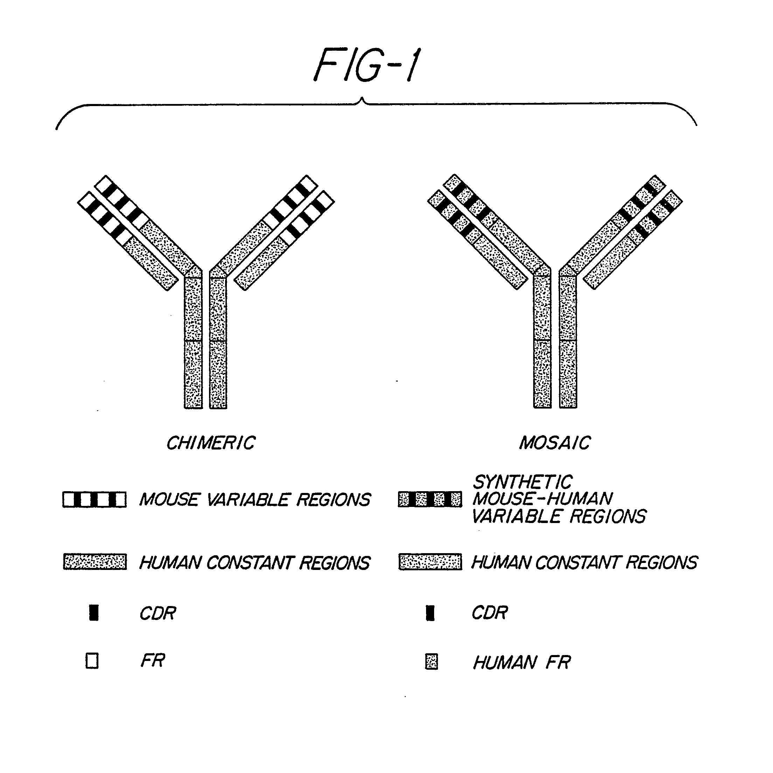

This embodiment of an antibody takes advantage of the fact that the variable region of both mouse and human immunoglobulins is comprised of four framework residues (FRs) which are interspersed with three complementarity determining residues (CDRs). The CDRs mediate antigen binding. Thus, in this approach, the mouse variable region genes coding for one or more of the CDRs are spliced into the human framework variable region and the resulting "mosaic" variable region may be spliced onto the human constant region forming a different type of chimeric antibody. Vectors containing these constructs may be transferred in to expressions systems as described above. A schematic for the traditional and mosaic forms of chimeric antibodies is shown in FIG. 1.

The ability to manipulate the gene sequences for any particular monoclonal antibody provides one further opportunity to design the antibody to precise specifications. By using appropriate recombinant DNA techniques, it is possible to create specific mutations in the nucleic acid sequence of the variable region gene. In some cases, this will lead to a change in the amino acid sequence of the immunoglobulin. This change, in turn, may lead to a conformational change in the structure of the immunoglobulin which may improve or diminish the binding capability or activities of the immunoglobulin. Thus, it may be possible to design two different immunoglobulins that have the same conformation and binding specificity but have different sequences.

This ability will effect not only the therapeutic format of the antibody but also will enable one to improve the diagnostic characteristics of the antibody. For example, one might use the chimeric or mosaic format to construct an antibody fragment (e.g., Fab' ) with different secondary properties but having the same or improved binding capabilities.

While the importance of the ability to change the conformation or structure of an immunoglobulin or to render it less immunogenic is unquestioned, the ability to make this changes requires identification of the specific sequence of the antibody in question. Thus, in order to make a chimeric or mosaic version of an antibody or to alter its conformation, the sequence must be known. Once it is known, improvements in its therapeutic capabilities then may be made.

## Summary of the Invention

This invention comprises the amino acid sequence for the monoclonal antibody Anti-Leu 3a variable region. It further comprises the amino acid sequence for each of the CDR portions of the Anti-Leu 3a variable region. A chimeric antibody having the amino acid sequence for the variable region and a mosaic antibody having one or more of the amino acid sequences for the CDR portion of the variable region also are claimed.

## Description of the Drawings

FIG. 1 comprises a schematic comparison of the chimeric and mosaic IgG antibodies. Chimeric antibodies consist of mouse variable regions

( )

and human constant regions

( ).

The mouse variable region contains the CDR (■) and the FR (□). Mosaic antibodies consist of synthetic mouse human variable regions

( )

containing mouse CDRs ( ■ ) and human FRs (▨) and human constant regions.

FIG. 2. comprises the nucleotide chain and deduced amino acid sequence of the cloned Anti-Leu 3a light chain variable region gene, 206 $V_x$.

FIG. 3 comprises the nucleotide and deduced amino acid sequence of the cloned Anti-Leu 3a heavy chain variable region gene, 316-$V_H$.

FIG. 4 comprises the nucleotide and predicted amino acid sequence of the mosaic Anti-Leu 3a light chain variable region gene, KOL/206-$V_L$.

FIG. 5 comprises the nucleotide and predicted amino acid sequence of the mosaic Anti-Leu 3a heavy chain variable region gene, KOL/316-$V_H$.

FIG. 6 comprises a schematic synthesis strategy for mosaic light and heavy chain variable regions, wherein FRs are shown by thin boxes, CDRs as thick boxes, restriction sites by arrows and over-lapping, single- stranded oligonucleotides are represented by solid lines below each gene.

FIG. 7 comprises partial DNA restriction maps of human kappa and human gamma 1 expression vectors containing Anti-Leu 3a variable region sequences wherein exons are indicated by open boxes, enhancer elements are shown as open circles, dominant selectable markers are represented by shaded boxes, and antibiotic resistance genes are depicted with broken lines.

For FIG.s 2-5, the deduced amino acid sequence is shown below the nucleotide sequence in three letter code. The sequence of the mature protein is capitalized while the leader peptide is in lower case letters. CDRs are boxed. DNA regulatory elements (e.g., Parslow box and TATA box) are highlighted in boldface letters. Donor and acceptor splice sites are underlined and splice junctions are indicated be an up arrow.

## Detailed Description of the Invention

Organization of mouse immunoglobulin genome has been well described by Honjo, Ann. Rev. Immunol., 1:499 (1983). Briefly, the immunoglobulin gene system comprises three separate loci of $L_x$, $L_\lambda$ and H (light and heavy) chain genes, each chain containing the variable (V) and constant (C) genes. The $L_x$, $L_\lambda$ and H chain genes are located on mouse chromosomes 6, 16 an 12 respectively and on are located on human chromosomes 2, 22 and 14 respectively. Within each of the genes, a variety of introns and exons may exist.

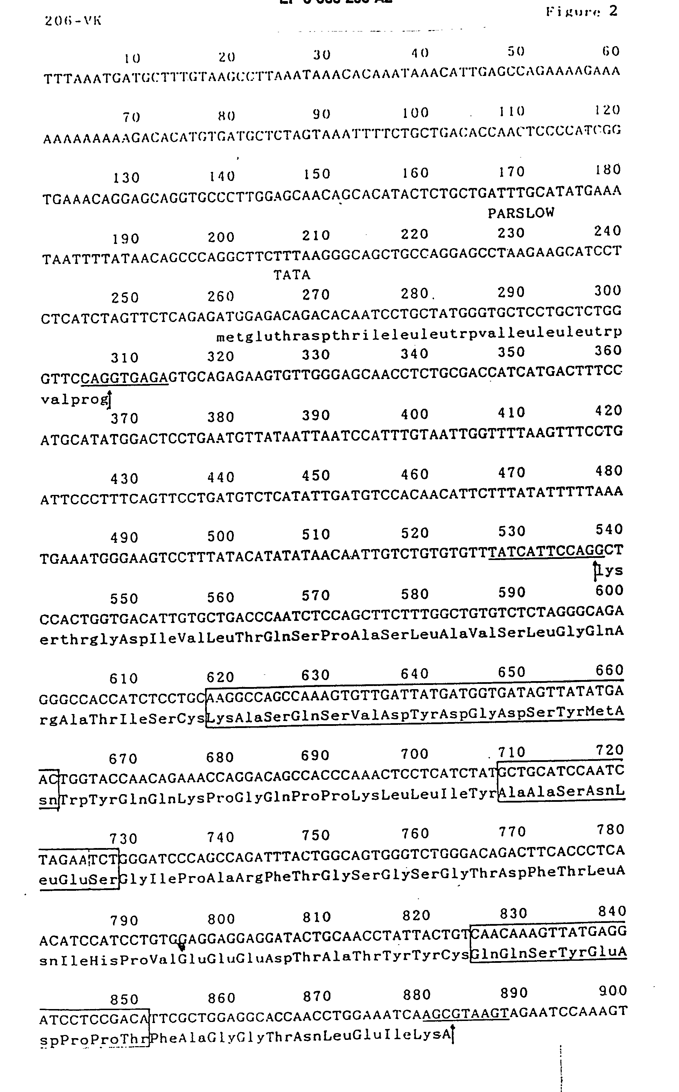

Referring to FIG. 2, the variable region sequence for the kappa light chain of Anti-Leu 3a was determined as follows. DNA containing the mouse variable region light chain gene was isolated from clone SK3 by screening a genomic library using hybridization probes. Genomic DNA was partially digested with MboI, ligated into the lambda replacement vectors EMBL3 or EMBL4, packaged, and amplified on an appropriate E. coli host. The libraries were screened using a ~0.85 Kb SacI HindIII probe from the mouse $J_x$-$C_x$ intron, or a ~0.70 Kb XbaI EcoRI enhancer probe from the mouse heavy chain intron.. Positive clones were characterized by Southern blotting, then were subcloned into the cloning vector pUC13.

Once the DNA for each of the light and heavy chain variable regions was isolated, it was sequenced by the chain termination method of Sanger et al., PNAS, 74:5463 (1977). Appropriate restriction fragments were subcloned into the sequencing vectors M13mp18, M13mp19 and PTZ18R, and the cloning vector pUC13. $^{35}$S- labelled templates were sequenced with Klenow fragment or AMV-reverse transcriptase.

The nucleotide sequence from positions 538 through 882 comprises the coding region for the Anti-Leu 3a $V_x$ chain. The amino acid sequence corresponding to the nucleotide sequence from positions 549 through 881 further comprises the structure of the variable region of the Anti-Leu 3a kappa light chain.

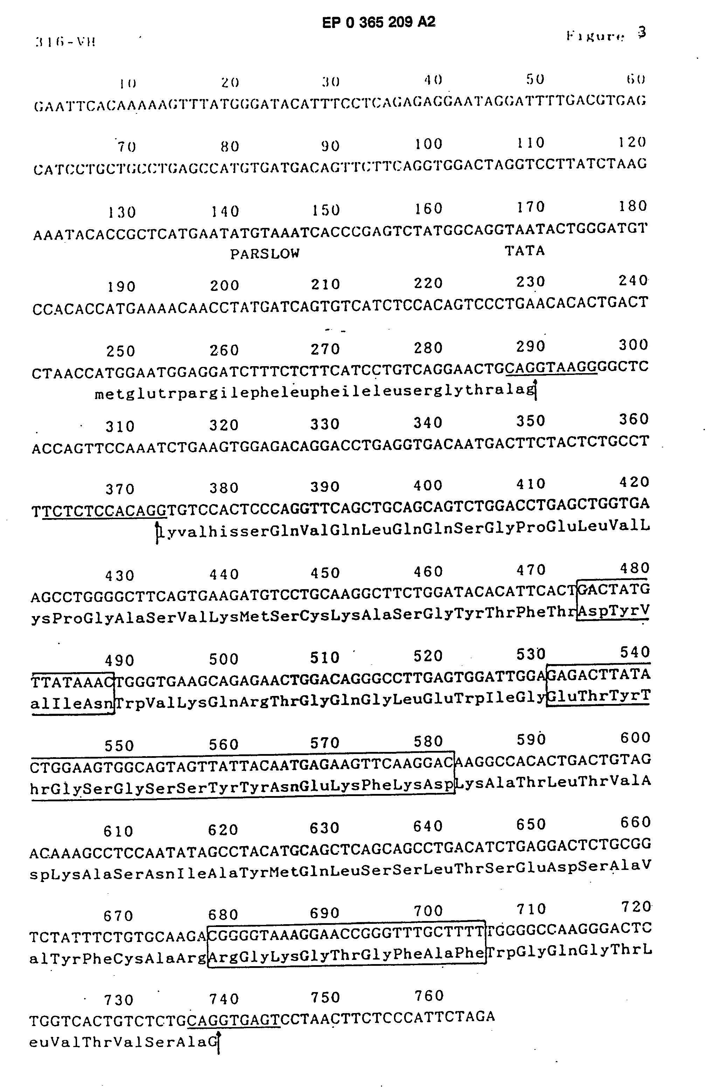

Referring to FIG. 3, the variable region sequence for the heavy chain of Anti-Leu 3a was determined as described above for the light chain except that appropriate restriction fragments were subcloned into the sequencing vectors M13mp18 and M13mp19 (Pharmacia), and the cloning vectors pUC12 and pUC13.

The nucleotide sequence from positions 373 through 738 comprises the coding region for the Anti-Leu 3a $V_H$ chain. The amino acid sequence corresponding to the nucleotide sequence from positions 384 through 737 further comprises the structure of the variable region of the Anti-Leu 3a heavy chain.

Once isolated, the nucleotide sequences for each or either of the variable region chains may be combined with nucleotide sequences for the human constant region. Once combined, the vector into which they are combined may be placed in an expression system for production of the chimeric antibody. Preferably, the method of Morrison and Oi as set forth in USSN 644,473 is used to construct the chimeric antibody; however, the methods set forth in USSN 483,457 may be used to construct the chimeric antibody in a prokaryotic system, such as E. coli.

Briefly, the 206-$V_x$ and 316-$V_H$ variable region genes were spliced to human kappa and human gamma 1 constant region genes and were inserted into the vectors pSV184neo and PSV2ΔHgpt respectively. The 206-$V_x$ gene was spliced to the human x gene at a unique HindIII site located in the large intron between the $J_x$ and $C_x$ exons. The 316-$V_H$ gene was spliced to the human gamma 1 gene at a unique EcoRI site located in the large intron between the $J_H$ and $C_H$ exons.

Transfection of a eukaryotic cell line was accomplished by the method described by Morrison et al., PNAS, 81:6851 (1984).

One Chimeric cell line that resulted from this transfection was named V23. This cell line produces a a chimeric mouse:human gamma 1 immunoglobulin that binds to the CD4 antigen on CD4[+] cells as confirmed by flow cytometric analyses and as confirmed by SDS-polyacrylamide gel electrophoresis of antigen antibody complexes from labelled cells.

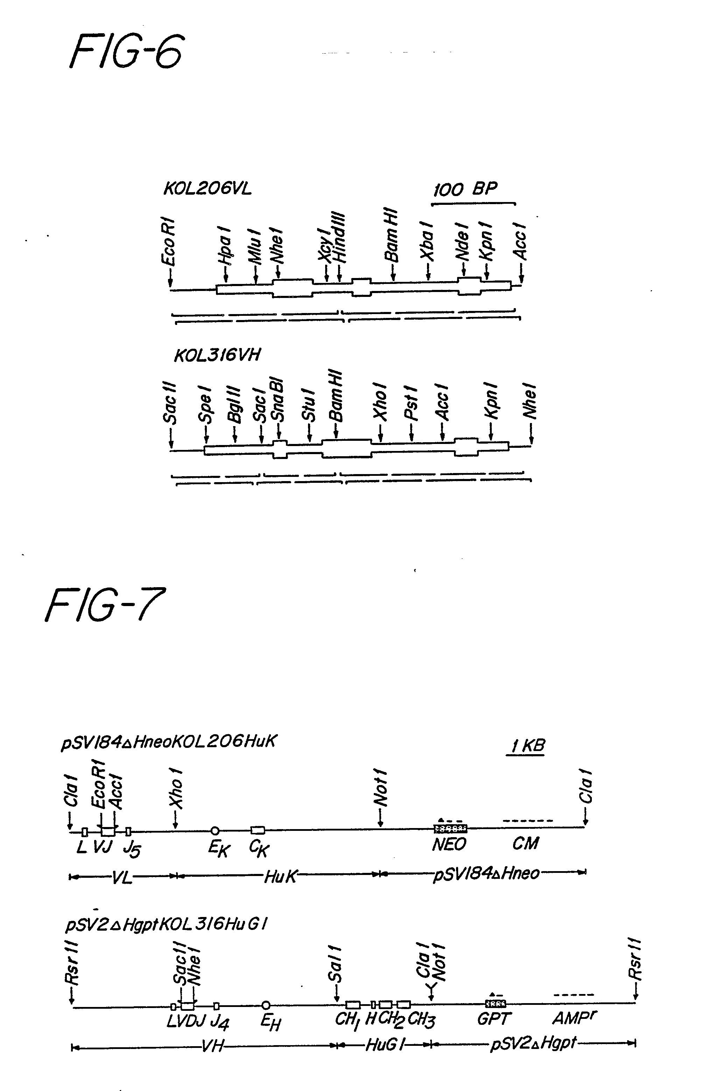

Referring to FIG.s 4 and 5, the nucleotide sequence of the $V_x$ and $V_H$ CDRs was determined next. The mosaic light chain variable region was synthesized from two 200 base-pair restriction fragments, each consisting of six oligonucleotides. The mosaic heavy chain variable region was synthesized from two 100 base-pair restriction fragments consisting of four oligonucleotides each and a 200 base-pair restriction fragment consisting of eight oligonucleotides. See FIG. 6.

Taking advantage of the degeneracy of the genetic code, unique restriction sites then were designed into each gene to facilitate CDR replacement by cassette mutagenesis. Thus, an altered amino acid sequence could be introduced by replacing the existing DNA sequence with a synthetic double-stranded restriction fragment. The human myeloma cell line KOL was used as a source of FRs for both the $V_x$ and $V_H$ chains even though it has a lambda light chain. KOL has been previously described by Bernstein et al., J. Mol. Biol., 112:535 (1977). The resulting mosaic genes then were subcloned into the bacterial cloning vectors PTZ18R and PTZ19R (Pharmacia) and were sequenced on both strands by the chain termination method described above.

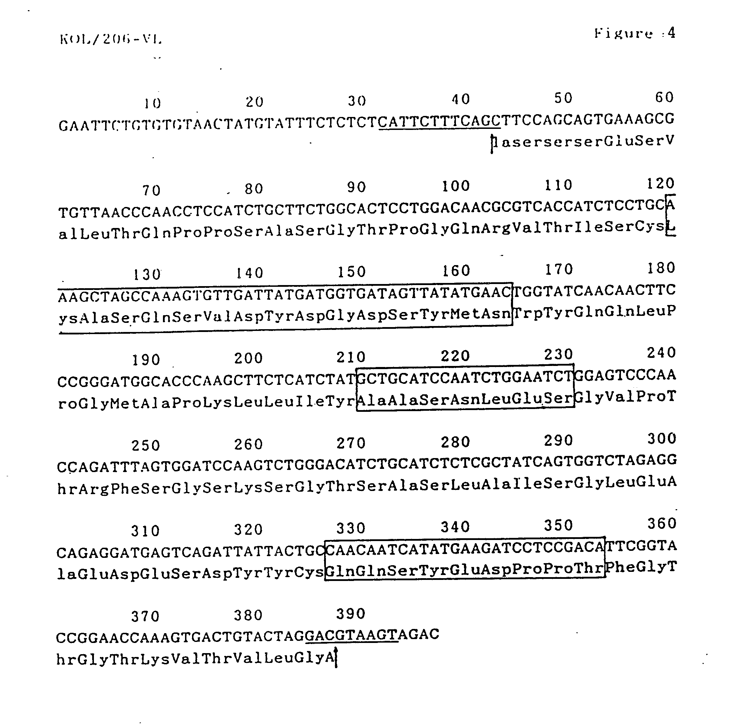

Referring to FIG. 4, the nucleotide sequence from positions 43 through 387 comprise the mosaic $V_x$ chain. The sequences from positions 120-164, 210-230 and 327-353 comprise the $CDR_1$, $CDR_2$ and $CDR_3$ regions respectively. The amino acid sequence for each CDR then was deduced from the nucleotide sequence.

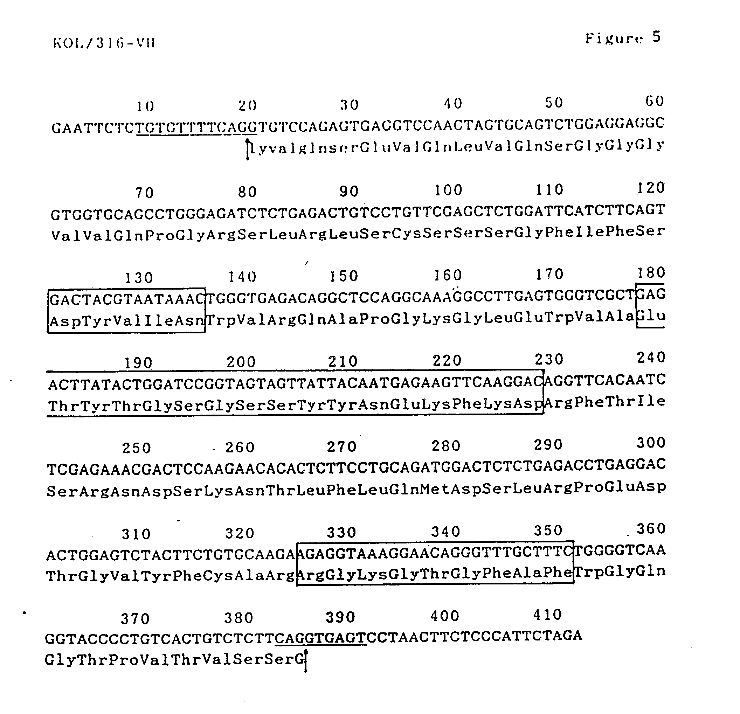

Referring to FIG. 5, the nucleotide sequence from positions 20 through 385 comprise the mosaic $V_H$ chain. The sequences from positions 121-136, 178-228 and 325-351 comprise the $CDR_1$, $CDR_2$ and $CDR_3$ regions respectively. The amino acid sequence for each CDR then was deduced from the nucleotide sequence.

As described above, expression vectors then were made to splice the mosaic constructs to the respective constant region genes. The vectors pSV184ΔHneo and PSV2ΔHgpt were used with the $V_x$ and $V_H$ mosaics respectively. Constant regions from KOL also were used as previously described. Each vector was modified by site-directed mutagenesis to permit insertion of the variable region mosaics. See FIG. 7.

Transfection of a eukaryotic cell line was accomplished by the method described in Oi and Morrison, BioTechniques, 4:214 (1986).

One mosaic cell line that resulted from this transfection was named 181-21. This cell line produces a mosaic mouse:human gamma 1 immunoglobulin that binds to the CD4 antigen on $CD4^+$ cells as confirmed by flow cytometric analyses and as confirmed by SDS-polyacrylamide gel electrophoresis of antigen-antibody complexes from labelled cells.

The V23 and 181-21 cell lines described above that produce the chimeric and mosaic Anti-Leu 3a antibodies have been deposited in the laboratory of Dr. Vernon Oi at the Becton Dickinson Monoclonal Center, Mountain View, California. It will be apparent to one skilled in the art that these are not the only possible combinations of Anti-Leu 3a derived sequences. Other combinations of sequences could include 1) having less than all CDRs in the construct be of mouse origin (e.g., combining $CDR_1$ in one or both chains from Anti-Leu 3a with $CDR_2$ and $CDR_3$ from another species), 2) having only the heavy or light chain be chimeric or mosaic (and thus having the other chain be totally of mouse origin) and 3) having a construct comprised of a mosaic chain and a chimeric chain. Other constructs may be made of different nucleotide sequences that have the structure and anti-CD4 binding function of Anti-Leu 3a but not do not necessarily have the entire Anti-Leu 3a sequence. Still other constructs may represent only a portion of the antibody (e.g., Fab'fragment ). Finally, other constructs could be made comprising anti-CD4 peptides that have both Anti-Leu 3a like binding functions (but lack an immunoglobulin structure) and the sequence of any of the variants described in FIG.s 2-5.

All publications and patent applications mentioned in this specification are indicative of the level of ordinary skill in the art to which this invention pertains. All publications and patent applications are herein incorporated by reference to the same extent as if each individual publication or patent application was specifically and individually indicated to be incorporated by reference.

It will be apparent to one of ordinary skill in the art that many changes and modifications can be made in the invention without departing from the spirit or scope of the appended claims.

## Claims

1. A chimeric antibody having a human constant region and having a mouse variable region comprising an amino acid sequence for a $V_x$ chain as described in FIG. 2.

2. A chimeric antibody having a human constant region and having a mouse variable region comprising an amino acid sequence for a $V_H$ chain as described in FIG. 3.

3. A chimeric antibody having a human constant region, and having a mouse variable region comprising an amino acid sequence for a $V_x$ chain as described in FIG. 2 and an amino acid sequence for a $V_H$ chain as described in FIG. 3.

4. An antibody having a mouse:human mosaic variable region comprising an amino acid sequence for a $V_x$ chain as described in FIG. 4.

5. An antibody having a mouse:human mosaic variable region comprising an amino acid sequence for a $V_H$ chain as described in FIG. 5.

6. An antibody having a mouse:human mosaic variable region comprising an amino acid sequence for a $V_x$ chain as described in FIG. 4 and an amino acid sequence for a $V_H$ chain as described in FIG. 5.

7. A chimeric antibody having a human constant region, and having a mouse:human mosaic variable region comprising an amino acid sequence for a $V_x$ chain as described in FIG. 4 and having an amino acid sequence for a $V_H$ chain as described in FIG. 5.

8. An antibody having a mouse:human mosaic variable region comprising at least one of the CDR amino acid sequences as described in FIG. 5.

9. An antibody having a mouse:human mosaic variable region comprising at least one of the CDR amino acid sequences as described in FIG. 6.

10. A nucleotide sequence for an anti-CD4 agent comprising any of the nucleotide sequences as described in any of FIG.s 2-5.

# FIG-1

CHIMERIC

MOSAIC

□□□□□ MOUSE VARIABLE REGIONS

▨▨▨▨ SYNTHETIC MOUSE-HUMAN VARIABLE REGIONS

▨▨▨▨ HUMAN CONSTANT REGIONS

▨▨▨▨ HUMAN CONSTANT REGIONS

▮ CDR

▮ CDR

□ FR

▨ HUMAN FR

206-VK                                                                 Figure 2

```
          10        20        30        40        50        60
TTTAAATGATGCTTTGTAAGCCTTAAATAAACACAAATAAACATTGAGCCAGAAAAGAAA

          70        80        90       100       110       120
AAAAAAAAAGACACATGTGATGCTCTAGTAAATTTTCTGCTGACACCAACTCCCCATCGG

         130       140       150       160       170       180
TGAAACAGGAGCAGGTGCCCTTGGAGCAACAGCACATACTCTGCTGATTTGCATATGAAA
                                                PARSLOW
         190       200       210       220       230       240
TAATTTTATAACAGCCCAGGCTTCTTTAAGGGCAGCTGCCAGGAGCCTAAGAAGCATCCT
                    TATA
         250       260       270       280       290       300
CTCATCTAGTTCTCAGAGATGGAGACAGACACAATCCTGCTATGGGTGCTCCTGCTCTGG
                  metgluthrasptthrileleuleutrpvalleuleuleutrp
         310       320       330       340       350       360
GTTCCAGGTGAGAGTGCAGAGAAGTGTTGGGAGCAACCTCTGCGACCATCATGACTTTCC
valprog
         370       380       390       400       410       420
ATGCATATGGACTCCTGAATGTTATAATTAATCCATTTGTAATTGGTTTTAAGTTTCCTG

         430       440       450       460       470       480
ATTCCCTTTCAGTTCCTGATGTCTCATATTGATGTCCACAACATTCTTTATATTTTTAAA

         490       500       510       520       530       540
TGAAATGGGAAGTCCTTTATACATATATAACAATTGTCTGTGTGTTTATCATTCCAGGCT
                                                           lys
         550       560       570       580       590       600
CCACTGGTGACATTGTGCTGACCCAATCTCCAGCTTCTTTGGCTGTGTCTCTAGGGCAGA
erthrglyAspIleValLeuThrGlnSerProAlaSerLeuAlaValSerLeuGlyGlnA

         610       620       630       640       650       660
GGGCCACCATCTCCTGCAAGGCCAGCCAAAGTGTTGATTATGATGGTGATAGTTATATGA
rgAlaThrIleSerCysLysAlaSerGlnSerValAspTyrAspGlyAspSerTyrMetA

         670       680       690       700       710       720
ACTGGTACCAACAGAAACCAGGACAGCCACCCAAACTCCTCATCTATGCTGCATCCAATC
snTrpTyrGlnGlnLysProGlyGlnProProLysLeuLeuIleTyrAlaAlaSerAsnL

         730       740       750       760       770       780
TAGAATCTGGGATCCCAGCCAGATTTACTGGCAGTGGGTCTGGGACAGACTTCACCCTCA
euGluSerGlyIleProAlaArgPheThrGlySerGlySerGlyThrAspPheThrLeuA

         790       800       810       820       830       840
ACATCCATCCTGTGGAGGAGGAGGATACTGCAACCTATTACTGTCAACAAAGTTATGAGG
snIleHisProValGluGluGluAspThrAlaThrTyrTyrCysGlnGlnSerTyrGluA

         850       860       870       880       890       900
ATCCTCCGACATTCGCTGGAGGCACCAACCTGGAAATCAAGCGTAAGTAGAATCCAAAGT
spProProThrPheAlaGlyGlyThrAsnLeuGluIleLysA
```

316-VH                                                              Figure 3

```
        10        20        30        40        50        60
GAATTCACAAAAAGTTTATGGGATACATTTCCTCAGAGAGGAATAGGATTTTGACGTGAG

        70        80        90       100       110       120
CATCCTGCTGCCTGAGCCATGTGATGACAGTTCTTCAGGTGGACTAGGTCCTTATCTAAG

       130       140       150       160       170       180
AAATACACCGCTCATGAATATGTAAATCACCCGAGTCTATGGCAGGTAATACTGGGATGT
                    PARSLOW                    TATA

       190       200       210       220       230       240
CCACACCATGAAAACAACCTATGATCAGTGTCATCTCCACAGTCCCTGAACACACTGACT

       250       260       270       280       290       300
CTAACCATGGAATGGAGGATCTTTCTCTTCATCCTGTCAGGAACTGCAGGTAAGGGGCTC
    metglutrpargilepheleupheileleuserglythralag

       310       320       330       340       350       360
ACCAGTTCCAAATCTGAAGTGGAGACAGGACCTGAGGTGACAATGACTTCTACTCTGCCT

       370       380       390       400       410       420
TTCTCTCCACAGGTGTCCACTCCCAGGTTCAGCTGCAGCAGTCTGGACCTGAGCTGGTGA
     lyvalhisserGlnValGlnLeuGlnGlnSerGlyProGluLeuValL

       430       440       450       460       470       480
AGCCTGGGGCTTCAGTGAAGATGTCCTGCAAGGCTTCTGGATACACATTCACTGACTATG
ysProGlyAlaSerValLysMetSerCysLysAlaSerGlyTyrThrPheThrAspTyrV

       490       500       510       520       530       540
TTATAAACTGGGTGAAGCAGAGAACTGGACAGGGCCTTGAGTGGATTGGAGAGACTTATA
alIleAsnTrpValLysGlnArgThrGlyGlnGlyLeuGluTrpIleGlyGluThrTyrT

       550       560       570       580       590       600
CTGGAAGTGGCAGTAGTTATTACAATGAGAAGTTCAAGGACAAGGCCACACTGACTGTAG
hrGlySerGlySerSerTyrTyrAsnGluLysPheLysAspLysAlaThrLeuThrValA

       610       620       630       640       650       660
ACAAAGCCTCCAATATAGCCTACATGCAGCTCAGCAGCCTGACATCTGAGGACTCTGCGG
spLysAlaSerAsnIleAlaTyrMetGlnLeuSerSerLeuThrSerGluAspSerAlaV

       670       680       690       700       710       720
TCTATTTCTGTGCAAGACGGGGTAAAGGAACCGGGTTTGCTTTTTGGGGCCAAGGGACTC
alTyrPheCysAlaArgArgGlyLysGlyThrGlyPheAlaPheTrpGlyGlnGlyThrL

       730       740       750       760
TGGTCACTGTCTCTGCAGGTGAGTCCTAACTTCTCCCATTCTAGA
euValThrValSerAlaG
```

KOL/206-VL                                                     Figure :4

```
        10        20        30        40        50        60
GAATTCTGTGTGTAACTATGTATTTCTCTCTCATTCTTTCAGCTTCCAGCAGTGAAAGCG
                                          ]aserserserGluSerV

        70      . 80        90       100       110       120
TGTTAACCCAACCTCCATCTGCTTCTGGCACTCCTGGACAACGCGTCACCATCTCCTGCA
alLeuThrGlnProProSerAlaSerGlyThrProGlyGlnArgValThrIleSerCysL

       130       140       150       160       170       180
AAGCTAGCCAAAGTGTTGATTATGATGGTGATAGTTATATGAACTGGTATCAACAACTTC
ysAlaSerGlnSerValAspTyrAspGlyAspSerTyrMetAsnTrpTyrGlnGlnLeuP

       190       200       210       220       230       240
CCGGGATGGCACCCAAGCTTCTCATCTATGCTGCATCCAATCTGGAATCTGGAGTCCCAA
roGlyMetAlaProLysLeuLeuIleTyrAlaAlaSerAsnLeuGluSerGlyValProT

       250       260       270       280       290       300
CCAGATTTAGTGGATCCAAGTCTGGGACATCTGCATCTCTCGCTATCAGTGGTCTAGAGG
hrArgPheSerGlySerLysSerGlyThrSerAlaSerLeuAlaIleSerGlyLeuGluA

       310       320       330       340       350       360
CAGAGGATGAGTCAGATTATTACTGCCAACAATCATATGAAGATCCTCCGACATTCGGTA
laGluAspGluSerAspTyrTyrCysGlnGlnSerTyrGluAspProProThrPheGlyT

       370       380       390
CCGGAACCAAAGTGACTGTACTAGGACGTAAGTAGAC
hrGlyThrLysValThrValLeuGlyA
```

KOL/316-VH                                                                                          Figure 5


```
          10         20         30         40         50         60
GAATTCTCTGTGTTTTCAGGTGTCCAGAGTGAGGTCCAACTAGTGCAGTCTGGAGGAGGC
              ↑yvalglnserGluValGlnLeuValGlnSerGlyGlyGly


          70         80         90        100        110        120
GTGGTGCAGCCTGGGAGATCTCTGAGACTGTCCTGTTCGAGCTCTGGATTCATCTTCAGT
ValValGlnProGlyArgSerLeuArgLeuSerCysSerSerSerGlyPheIlePheSer


         130        140        150        160        170        180
GACTACGTAATAAACTGGGTGAGACAGGCTCCAGGCAAAGGCCTTGAGTGGGTCGCTGAG
AspTyrValIleAsnTrpValArgGlnAlaProGlyLysGlyLeuGluTrpValAlaGlu


         190        200        210        220        230        240
ACTTATACTGGATCCGGTAGTAGTTATTACAATGAGAAGTTCAAGGACAGGTTCACAATC
ThrTyrThrGlySerGlySerSerTyrTyrAsnGluLysPheLysAspArgPheThrIle


         250        260        270        280        290        300
TCGAGAAACGACTCCAAGAACACACTCTTCCTGCAGATGGACTCTCTGAGACCTGAGGAC
SerArgAsnAspSerLysAsnThrLeuPheLeuGlnMetAspSerLeuArgProGluAsp


         310        320        330        340        350        360
ACTGGAGTCTACTTCTGTGCAAGAAGAGGTAAAGGAACAGGGTTTGCTTTCTGGGGTCAA
ThrGlyValTyrPheCysAlaArgArgGlyLysGlyThrGlyPheAlaPheTrpGlyGln


         370        380        390        400        410
GGTACCCCTGTCACTGTCTCTTCAGGTGAGTCCTAACTTCTCCCATTCTAGA
GlyThrProValThrValSerSerG
```

# FIG-6

KOL206VL                    100 BP

KOL316VH

# FIG-7

pSVI84ΔHneoKOL206HuK          1 KB

pSV2ΔHgptKOL316HuGI